(19) Europäisches Patentamt
European Patent Office
Office européen des brevets

(11) **EP 1 421 382 B1**

(12) **EUROPEAN PATENT SPECIFICATION**

(45) Date of publication and mention
of the grant of the patent:
**15.08.2012 Bulletin 2012/33**

(51) Int Cl.:
*G01N 33/50* (2006.01)   *G01R 33/12* (2006.01)

(21) Application number: **02733720.3**

(22) Date of filing: **17.05.2002**

(86) International application number:
**PCT/SE2002/000964**

(87) International publication number:
**WO 2003/019188 (06.03.2003 Gazette 2003/10)**

(54) **METHDO AND ARRANGEMENT FOR ANALYZING SUBSTANCES**

VERFAHREN UND ANORDNUNG ZUR ANALYSE VON SUBSTANZEN

PROCEDE ET ARRANGEMENT D'ANALYSE DE SUBSTANCES

(84) Designated Contracting States:
**AT BE CH CY DE DK ES FI FR GB GR IE IT LI LU MC NL PT SE TR**

(30) Priority: **31.08.2001 SE 0102926**
**31.08.2001 US 316040 P**

(43) Date of publication of application:
**26.05.2004 Bulletin 2004/22**

(73) Proprietor: **IMEGO AB**
**411 33 Göteborg (SE)**

(72) Inventors:
• **MINCHOLE, Ana**
**E-50006 Zaragoza (ES)**

• **PRIETO-ASTALAN, Andrea**
**S-422 48 Göteborg (SE)**
• **JOHANSSON, Christer**
**S-413 24 Göteborg (SE)**
• **LARSSON, Kerstin**
**S-416 56 Göteborg (SE)**
• **KROZEN, Anatol**
**S-413 17 Göteborg (SE)**

(74) Representative: **Hammond, Andrew David et al**
**Valea AB**
**Lindholmspiren 5**
**417 56 Göteborg (SE)**

(56) References cited:
**US-A- 6 027 946**

## Description

TECHNICAL FIELD

The present invention relates to an arrangement for detecting changes of a magnetic response with at least one magnetic particle provided with an external layer in a carrier fluid, thus the method comprises using a measuring method comprising measuring the characteristic rotation time of said magnetic particle with respect to said external layer, which measuring method Involves measuring Brownian relaxation in said carrier fluid under the influence of an external alternating magnetic field.

BACKGROUND OF THE INVENTION

Magnetic spherical particles with a diameter of less than about 20 nm are magnetic mono domains both In a magnetic field and in the zero field. A particle being a magnetic mono domain means that the particle only contains one magnetization direction.

Depending on the size, geometry, temperature, measurement time, magnetic field and material of the particles, they can either be thermal blocked or super-paramagnetic. The direction of the magnetization for thermal blocked particles are oriented In a specific direction In the magnetic particle in proportion to the crystallographic orientation of the particle, and "locked" to this direction, meanwhile studying the particle system. Under Influence of an external magnetic field, the entire particle physical rotates so that their magnetization directions gradually partly coincide with the direction of the external added field.

Small magnetic particles can be manufactured In a number of materials, for example magnetize ($Fe_3O_4$), Maghemite (y- $Fe_2O_4$), cobalt doped iron oxide or cobalt Iron oxide ($CoFe_2O_4$). Other magnetic materials, specially (but not exclusively) rare earth metals (for example ytterbium or neodymium), their alloys or compounds containing rare earth metals, or doped magnetic (element) substances can also be possible. The sizes of the particles can be produced from about 3 nm to about 30 nm. The final size In this process depends on a number of different parameters during the manufacturing,

Magnetization In small particles can relax In two different ways, via Néel relaxation or on the other hand via Brownian relaxation. These relaxation phenomena are related to particles with a magnetic arranged structure. They should not be mistaken for nuclear magnetic (NMR) resonance phenomenon's, the latter describes resonances within the atomic nucleus. The latter resonance phenomena have resonance frequencies typically within the GHz-range unlike resonance frequencies for the phenomenon considered In this patent, which is In the Interval from few Hz till some MHz.

Néel relaxation

In Néel relaxation the magnetization In the particle relax without the particle physical rotating (no thermal blocking). The relaxation period for this kind of relaxation strongly depends on size, temperature, material and (at high particle concentrations) on the magnetic interaction between the particles. For this relaxation being available the magnetization direction In the particle has to change direction fast In time, the particles have to be super-paramagnetlc. Néel relaxation period in the zero field can be described according the equation below:

$$\tau_N = \tau_0 e^{\frac{KV}{kT}}$$

wherein $t_0$ is a characteristic relaxation period, $K$ is the magnetic anisotropic constant, $V$ magnetic particle volume, $k$ Boltzman's constant and $T$ temperature.

Brownian relaxation

In the Brownian relaxation, the magnetization-direction rotates when the particle physically rotates. For this relaxation being available the magnetization has to be locked in a specific direction In the particle, the particle has to be thermal blocked. The relaxation period for Brownian relaxation depends on hydrodynamic particle volume, viscosity of the carrier fluid wherein the particles are dispersed in, connection between the surface of the particle and the fluid layer nearest it's surface (Hydrophobic and hydrophilic respectively). The Brownian equation can approximately be described according to the equation below:

$$\tau_B = \frac{3V_H \eta}{kT}$$

wherein $V_H$ Is the hydro dynamical volume for the total particle (inclusive of the polymer layer), $\eta$ viscosity for the surrounding carrier fluid, $k$ Boltzmann's constant and $T$ is the temperature. In the derivation above a perfect wetting (hydrophilic) has been assumed and a constant rotation speed (the initial approximation has been neglected).

The Brownian relaxation period accordingly depends on the (effective) size of the particle and the environmental effect on the particle. To discern If a particle shows Brownian relaxation or Kneeling relaxation you can among other things study whether external Influences (for example an other fluid viscosity, temperature changes, applied static magnetic field) changes the relaxation period.

You can also study the phenomenon in the frequency domain, when it concerns determining the resonance frequencies regarding the particle system. These can be obtained for example by means of AC-suspetometri (for Brownian relaxation some Hz till kHz region and for Néel relaxation typically in the MHz region).

Apparently above a Brownian movement (Brownian relaxation) depends among other things on the volume of the particle: the lager particle the longer relaxation period that Is, the smaller the movement of the particle gets. Relaxations periods for particles lager than about 1 $\mu$m are much longer than 1 second, which In practice means a negligible movement. These particles can though even be used at detection. Larger particles can however show other types of relaxations wherein the inertia of the particles and viscosity elastic characteristics of the carrier fluid must be included for a sufficient data interpretation

Frequency susceptibility

The magnetization for a particle system In an alternating magnetic field can be described according to:

$$M = \chi H = (\chi^{'} - j\chi^{"})H$$

wherein $M$ is the magnetization, $H$ the alternating external magnetic field, $\chi$ the frequency dependent complex susceptibility consisting of an In phase component (real part), $\chi'$, and one out of phase component (Imaginary part), $\chi'$. The In phase and the out of phase components for a magnetic particle system can approximately be described as:

$$\chi^{'} = \frac{\chi_0}{1 + \left(2\pi f\tau\right)^2}$$

$$\chi^{"} = \frac{\chi_0\left(2\pi f\tau\right)}{1 + \left(2\pi f\tau\right)^2}$$

Wherein $\chi_0$ Is the DC value of the susceptibility and $\tau$ Is the relaxation period for magnetic relaxation.

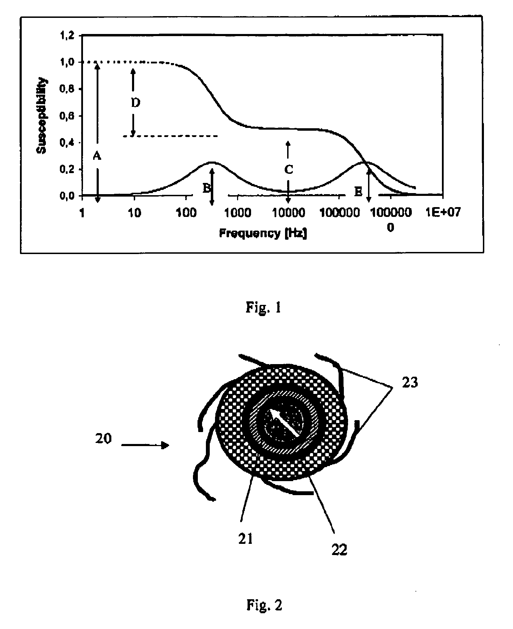

Assuming a particle system with varying particle sizes wherein some of the particles go through Brownian relaxation (the larger particles) and some Néel relaxation (the smaller particles) you obtain a magnetic response contribute from both the relaxation processes depending on the frequency range AC field. Fig. 1 shows schematically the total magnetic response as a function of the frequency for the particle system that shows both Brownian and Néel relaxation. The upper graph (dashed line) in the figure Is the real part of the susceptibility and the lower graph (continuous line) is the imaginary part of the susceptibility. The maximum for the imaginary part at lower frequencies Is from the Brownian relaxation and the maximum at high frequencies is from the Néel relaxation. The total magnetic response is the sum of the contributions from both the processes for both real and imaginary part of the susceptibility.

For this application only the Brownian relation is interesting, therefore the discussion is concentrated at these lower frequencies.

For a particle system with particles showing Brownian relaxation with only one hydrodynamic volume you obtain a maximum In the out of phase component ($\chi$", the imaginary part of the complex susceptibility) at a frequency according to:

$$f_{max} = \frac{1}{2\pi\tau_B} = \frac{kT}{6\pi V_H \eta}$$

Round this frequency, $f_{max}$, the real part of the susceptibility, $\chi'$, will decline very much white the imaginary part of the susceptibility, $\chi$", will show a maximum. The value of $\chi$" at the maximum (B In the figure 1) is among other things a measure of the number of particles that goes through Brownian relaxation while the level of the magnetic response for $\chi'$ (C In figure 1) after the maximum In $\chi$" is a measure of the total number of particles that still magnetic can follow the applied AC field (In this case particles that goes through Néel relaxation). At sufficient low frequencies all particles can magnetic follow the AC field, that Is, the real part of the susceptibility at these low frequencies (A In figure 1) Is a measure of the total number of particles. The contribution from the Brownian particles can then be quantified as the difference between the total contribute, A and the Néel contribute, C (D In figure 1). At higher frequencies, a new maximum is obtained in $\chi$" as a result of the Néel relaxation (E In figure 1). The comparison between these two values Is therefore a measure of the concentration of particles In a sample that goes through the Brownian relaxation, which is of Interest for this application. The width of the maximum of $\chi$",$\delta$ $f_{max}$ (and the rate of the subside of $\chi'$) is a measure of energy dissipation due to the fluids repercussion on the particles (the friction). The friction vary with (above all) the spreading In the hydrodynamic volume between the particles as a particle population in a sample can show., but depends partly also on statistical (temperature dependent) fluctuations.

Through measuring susceptibility, the Brownian relaxation and the energy dissipation, one could determine the total concentration of particles, the degree of particles that goes through Brownian relaxation In this particle population, the medium size of a particle In a carrier fluid and the spreading In particle volumes.

Magnetic particles have earlier been used as carrier of bio molecules or antibodies for measuring changes in their magnetic response. In these methods, the particles are either bound to a fixed surface or the particles are aggregated. One has measured how the magnetic resonance decrease with time [6] after that the particle system Is magnetized or the magnetic response has been measured when an external magnetic field is applied over the magnetic particles [8]. In these measurements, one has been able to part between the Néel relaxation and the Brownian relaxation. The measurements are done with a totally different technique then what is the case for the present invention, so called SQUID-technique that requires cryofluids and advanced electronics has been used. Grossmann et al, ref. 6, also uses antibody cased magnetic nanoparticles for determining specific target molecules, but combines this with the SQUID technology, that Is, with a supra conducting detector.

There are three substantially differences between the procedure according to present invention and the above mentioned methods:

(I) the physical principles behind the measurements according to the invention are different from earlier performances when others have chosen to measure in time/period domains Instead of in frequency domains as shown in this case, and also that the it Is necessary to "pre-magnetizes" the particle system,

(II) The method of measurement that many uses for measuring Is constructed from a, certainly very sensitive, but expensive and complicated technology, - namely the SQUID technology.

(III) The invention is based on that the agglomeration of the particles Is avoided, this is accomplished through providing the particles with a surface with characteristics so that agglomerations isn't formed. For example, monoclodical antibodies reacting specifically with the substance to be analyzed can cover the surface of the particles. According to known technique bio molecules with multiple ways of bonding have been analyzed.

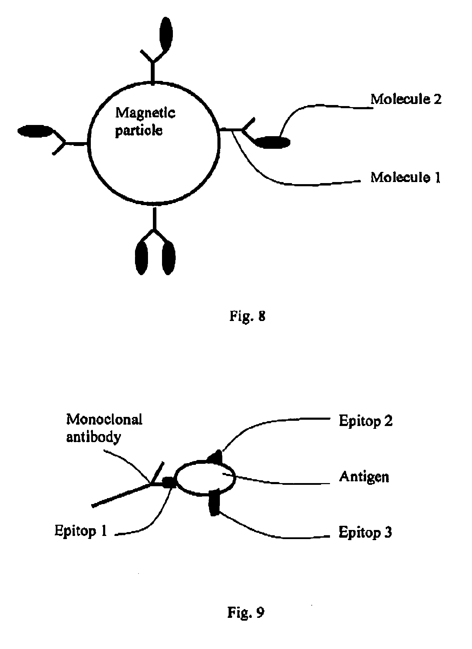

Kötitz et al, ref 7, has also been studying the Brownian relaxation in system of magnetic nanoparticles. They have been using magnetic particles covered with blotine. To this system they have added different amounts of avidin. When avidin has 4 bonding places to blotine, avidin including agglomerate is created. In the present method, the molecule 1 and molecule 2 are chosen in such a way that no agglomerate is created. It can for example be monoclonal antibodies (molecule 1) that are bond to the magnetic particle. This monoclonal antibody bonds to a specific etipope on the target molecule, which leads to prevention of agglomerate (fig. 9).

Yet, another thing that distinguish the method according to the invention from similar methods is that In this case how the frequency dependent of the magnetic response Is changed at different measurement frequencies with a relatively simple measuring set up. What further distinguishes the present method Is that according to the invention different bio molecules or antibodies are bond till the particle surface that changes the hydrodynamic volume. According to earlier methods particles are bond to a fixed surface or the particles are aggregated.

US 6,027,946 relates to a method and process for qualitative and/or quantitative detection of analytes In liquid phase or solid phase, comprising labeling the analytes with ferromagnetic colloidal particles or ferrimagnetic colloidal particles, and determining relaxation of magnetization of said labeled analytes as a qualitative or quantitative measurement of the analyte. However, this document differs In that the according to the present invention Brownian relaxation is measured under the influence of an external alternating magnetic field and upon modification of the effective volume of the particle or its interaction with the carrier fluid a hydromagnetic volume of the particle changes, resulting in change of the frequency.

BRIEF DESCRIPTION OF THE INVENTION

The invention relates to detecting changes in the magnetic response of the magnetic particles that shows the Brownian relaxation in a carrier fluid (for example water or a suitable buffer fluid, or another fluid suitable for the blo molecules that are the final target for the detection) under Influence of an external AC-magnetic field. At the modification of the efficient volume of the particles or their interaction with the surrounding fluid, for example when bio molecules or antibodies are bond on their surfaces, the hydro dynamic volume of respective particles will be changes (increase) that means a change (reduction) of the frequency, $f_{max}$, wherein the out of phase component of the magnetic susceptibility are having it's maximum.

Hence, the initially mentioned method comprises use of a method further Involves that upon modification of the effective volume of the particle or Its interaction with the carrier fluid a hydrodynamic volume of the particle changes, which implies a change of the frequency where an out of phase component of the magnetic susceptibility has Its maximum. The measurement is actually a relative measurement, changes In a modified particle system are compared with an original system. At least two sample containers and two detector coils are used for the measurement. Preferably, an oscillator circuit at a frequency is used, that Is the resonance frequency, wherein detector coil are placed as a frequency-determining element In the oscillator circuit so that they are out of phase with each other. The frequency change and/or effect or the amplitude of the oscillations from the oscillation circuit over the coils is therefore measured.

An external oscillator/frequency generator can be arranged, at which the coils are In an alternating bridge so that the difference between both detector coils are measured, and that the phase difference between the output current and/or voltage of the frequency generator and a current/voltage over the bridge is measured. In this case an amplitude difference between the oscillator output current/voltage can be measured and compared with amplitude of the current/voltage in the bridge. The measurement is accomplished at one or several different frequencies.

A noise source can be used as well and that the response of the system can be analyzed by means of a FFT (Fast Fourier Transform) analysis of an output signal,

According to one embodiment, the signal difference Is set to zero between the coils, which is done through mechanically adjusting position of the sample containers respectively, alternatively change the position of the detection coils respectively so that the difference signal is minimized. The zero setting can be done through minimizing the signal through adding a determined amount of a magnetic substance In one of the spaces wherein the sample containers are placed, so that the substance creates an extra contribution to the original signal that therefore can be set to zero. The magnetic substance shows substantially zero magnetic loss (Imaginary part=0) and that the real part of the susceptibility Is constant in the examined frequency range.

The method Is preferably but not exclusively used in analysis instruments for analyzing different bio molecules or other molecules In fluid. the molecules, comprises one or several proteins in a fluid solution, like blood, blood plasma, serum or urine. The analysis (molecule 2) can be connected to the particle through interaction with a second molecule (molecule 1), which Is connected to the particle before the analysis starts. Molecules that can be Integrated specific which each other can comprise one or several of antibody - antigen, receptor - hormone, two complementary single strings of DNA and enzyme - substrate / enzyme - inhibitor.

According to a preferred embodiment, the surface of the magnetic particle Is modified through covering the surface with one or several of dextrane, with alkanethiols with suitable end groups or with some peptides. The dextrane surface (or another suitable intermediate layer) can then a first molecule, for example an antibody, be bond by means of for example cyan bromide activation or carboxyl acid activation.

The invention also relates to an arrangement for performance of a method for detection of changes In the magnetic response of at least one magnetic particle provided with an external layer In a carrier fluid, which method comprises measurements of the magnetic particles characteristic rotation period with respect to the agitation of the external layer. The arrangement comprises at least two substantially identically detection coils connected to detection electronics and sample containers for absorbing carrier fluid. An excitation coil can surround the detection coils and sample containers for generation of a homogeneous magnetic field at the sample container. According to one embodiment when the excitation coil, measurement coils and also sample container are placed concentric and adjusted round Its vertical center axis. The arrangement can furthermore comprise an oscillator system wherein the detection coils constitutes the frequency-determining element In an oscillator circuit. The coils are arranged In the oscillator return coil. The coils that surround the samples respectively are electrically phase shifted versus each other so that the resonance frequency Is determined from the difference between the Inductance and the resistance respectively of the coil. The coils are placed In an AC-bridge. Additionally, an operation amplifier can be arranged to subtract two voltages from each other.

The arrangement comprises a phase locking circuit in one embodiment. In a second embodiment the arrangement comprises oscillator/frequency generator signal to generate period variable current to excite the coils by means of white noise. Frequency depending Information Is received through an FFT-filtering of the response.

The inventions also relates to a method of determining an amount of molecules in a carrier fluid containing magnetic particles, where the determination may comprise the steps of:

A. providing the magnetic particles with a layer, which Inter-/reacts with the substance to be analyzed,

B. mixing the magnetic particles with a sample to be analyzed with respect to molecules,

C. filling a sample container with the fluid being prepared according to B,

D. placing sample container In the detection system,

E. applying an external measure field over the sample with a certain amplitude and frequency,

F. measuring up the magnetic response (both In phase and out of phase components) at this frequency,

G. changing frequency and executing the measurement according to D or E,

H. analyzing the result through determining a Brownian relaxation period from In phase and out of phase components through using data In the examined frequency Interval.

The method further involves determining the frequency shift (for same value of In phase and out of phase components) at different frequencies. The molecule consists of a bio molecule.

DESCRIPTION OF THE DRAWING

In the following the invention will be described with respect to some embodiments and with references to the enclosing drawings, In which:

Fig. 1      shows the magnetic response as a function of frequency for a particle system showing both Brownian and Néel relaxation,

Fig. 2      shows schematically a section through a rotating magnetic particle with suitable intermediate layers and bio molecules,

Fig. 3      shows how in phase and out of phase components of the magnetic susceptibility vary with the frequency at room temperature for two different hydro dynamic diameters,

Fig. 4      shows the equivalent circuit of a coil,

Fig. 5      shows schematically a section through an exemplary measure system, according to the Invention,

Fig. 6      shows adjustment of the measure system, according to the invention by means of adding a magnetic material showing $\chi'$ = constant and $\chi''$ = 0, In the frequency Interval used while measuring the Brownlan relaxation,

Fig. 7      shows schematically an alternative detection circuit (differential measurement without excitation coil), according to the invention,

Fig. 8      shows schematically an application, according to the invention, and

Fig. 9      shows a monoclonal antibody Integrating with only one epitope on an antigen.

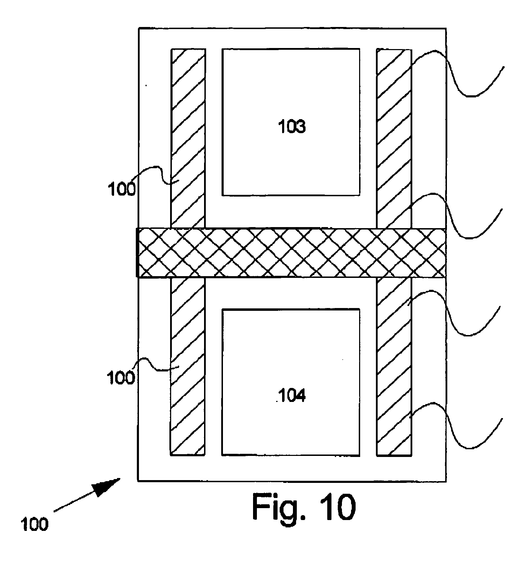

Fig. 10      Is a schematic cross-section through a second exemplary measurement system, according to the invention,

Fig. 11      is a schematic second alternative detection circuit (differential measuring without excitation coils), according to the invention,

Fig. 12      is an alternative to Fig. 7 coupling of coils. Here, they are coupled in parallel with respect to each other in a suitable circuit.

DESCRIPTION OF THE INVENTION

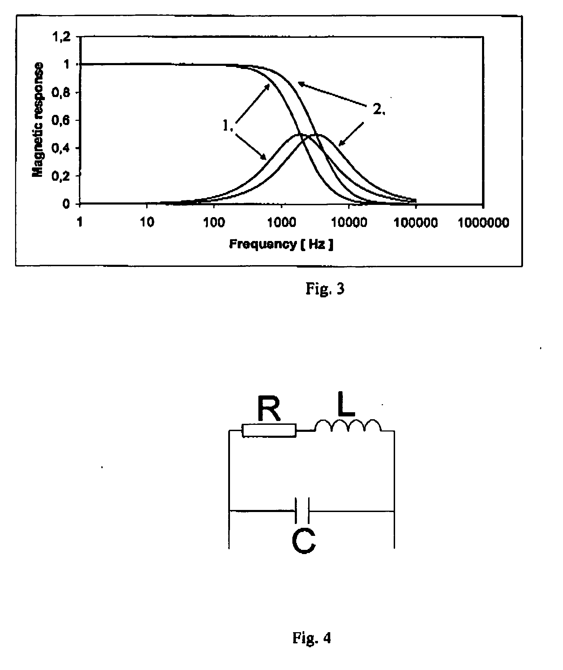

Figure 3 shows how In phase and out of phase components of the magnetic susceptibility vary with frequency at room temperature for two different hydro dynamic diameters, 50 nm (the graphs 2) and 60 nm (the graphs 1) when the particles goes through Brownian relaxation. The particles are dispersed In water. Out of phase components for the particles respectively shows a maximum at that frequency corresponding to the Brownian relaxation period while the In phase components subsides at that frequency.

In figure 3 is also shown how the magnetic response will change In the frequency plane at different hydrodynamic volumes. I these calculations, thermal blocked magnetic particles and only one particle size have been assumed (In a real particle system we always have a certain size distribution), which will result In a somewhat wider magnetic response In the frequency plane but it will not effect the present method. In the figure, one can see that when the hydrodynamic diameter increases, the magnetic response will shift downwards I the frequency. By measuring this frequency shift, one can determine, for example If a certain biomolecule has been attached to the surface (the hydrodynamic has thus Increased) or If the Interconnection between different biomolecules has occurred. As the frequency shift depends on the size of the blomolecules and the characteristic of their interaction with the surrounding fluid, It Is also possible to determine the relative concentrations of respective biomolecules or antibodies by studying the extent of the frequency shift.

A known procedure is to detect both $\chi'$ and $\chi''$ over a broad frequency Interval from some Hz to nearly some MHz for different (surface-) modifications and comparing these with each other (see figure 1 and 3) via a subsequent treatment of the collected data. Another, often used method of characterizing Brownian movement at a particle system is to study the response of the particles to alternating magnetic field In the time domain: so-called relaxation time measurements. As the invention handles measurements In the frequency domain, we will avoid closer description of measurement method at relaxation time measurements.

If the requirement Is to examine the effect of particle modification (-modifications) the viscosity of the fluid should remain constant. Viscosity changes also changes the Brownian movement of the particles, and changes $\chi'$ and $\chi''$ frequency dependent. Influence of viscosity changes can therefore be hard to separate from contributions caused by among other thing particle modifications. On the other hand the effect can be used for comparing different fluids viscosities when using Identical particles but changes the fluid In question.

One method is to focus on the detection $\chi'$ and $\chi''$ at only one frequency, $f_{max}$, and at the same time determine $\delta f_{max}$, or round a few discreet frequency values. If required a given particle system can be characterized separately, for example with respect to Brownian relaxation degree or the spreading size.

To make these methods work the particles must have a thermally blocked magnetic core (magnetic particle volume) which limit particle sizes and the magnetic anistropine of he magnetic core.

A typical particle system suitable to use for this method is a particle with a magnetic core made of magnetite or maghemite with a diameter of about 20 nm. There are also other materials with particles showing thermal blocked magnetization, for example Co doped ferric oxide or $CoFe_2O_4$ with a size of about 10 nm - 15 nm, possibly rare earth metals, and other.

In many applications, especially they considered below, the magnetic core Is covered with an external layer, for example a polymer like polyacrylamide or dextrane. Other covering materials can of course also occur, for example metal layers (like Au), other polymer, specific chemical compounds like silanes or thioles, and so on. It is often suitable to choose the thickness of the layer so that the total particle diameter varies from about 25 nm up to 1 μm (or higher).

To receive a percentage frequency transmission at particle modifications as large as possible relatively small particles (about 50 nm) are used. It Is assumed that If total sizes (diameters) from about 50 nm to 1 μm are used large enough percentage frequency changes are received with our method.

Fig. 2 illustrates a magnetic core 20 covered with 2 extra layers 21, 22 that are rotating anticlockwise. The thick black lines shown in the figure between the different layers Illustrates the intermediate surface material that can be separated from the material of which the bulk of the layer consists. To the external layer 22 long and thin bio molecules 23 have been attached. The sketch of the particle Illustrates a further important condition that the particle preparation should comply with: the material in the different layers are chosen so that the different layers are anchored to each other enough strong (the bonding enthalpine of the intermediate layer Is high) so that they are prevented from rotating in proportion to each other when the external magnetic field is applied to the particle.

A usually used detection method Is to detect *the change* in Induced voltage for a double flushing system (detection flushing system) positioned in an excitation coil. The sample is placed In one of the detection coils. In this case a lock-in amplifying technique Is used to measure the signal from the sample. This method is very sensitive and used In most commercial AC susceptometers. The frequency Interval Is typically from about 0,01 Hz up to 10 kHz. It's hard to measure at higher frequencies with this measuring system. It's possible to measure up to slightly higher frequencies, for example 60 kHz, but this requires a specific designed measurement system. To measure the susceptibility at yet higher frequencies, for example up to 10 MHz, a method based on detection of changes In Inductance and resistance can be used for a toroid coil system with a soft magnetic material (for example mu-metal or some kind of ferrite material If high measuring frequencies are used). The sample Is then placed In a thin gap In the magnetic toroid and one measures the circuit parameters of the toroid when the gap Is empty and after placing the sample in the gap, respectively.

Common for all these methods is that one can represent characteristics of a spiral wounded coil with an equivalent electric circuit consisting of an inductance, L, In series with a resistance, R, (connected to a capacitance, C, In parallel with these. The capacitance depends on the electrical isolation of the thread and can often be neglected at lower frequencies, see figure 4) wherein the resistance and the inductance of the circuit can be changed when a magnetic sample is placed in the coil.

If a variable (AC) current I(ωt) (In phase with the AC magnetic field) is floating in the circuit It will induce a complex voltage which real part is in phase with the current while the imaginary part is out of phase in proportion to I(ωt).

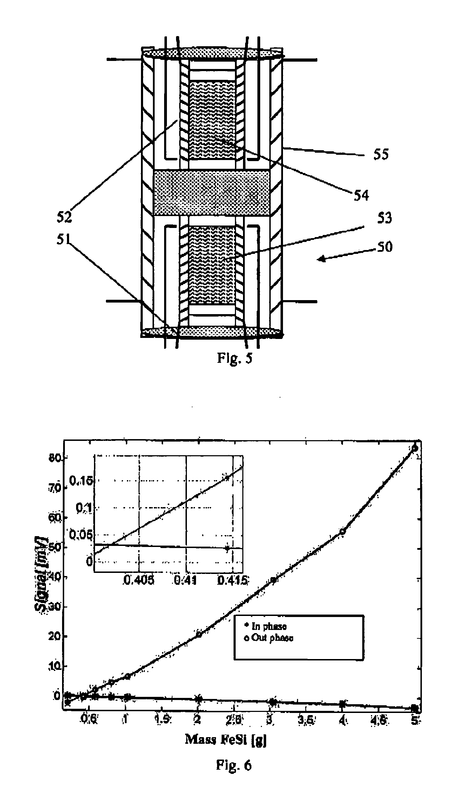

Since, In the first place *differences* in the susceptibility are to be determined in the susceptibility that occurs at different particle preparations (or compare viscosities of two different fluids) a measure system in constructed differently than usual used measuring systems. The measuring system 50, shown schematically in Fig. 5, consists of two identical detection coils 51, 52, surrounding two identical sample containers 53, 54 similar to commercially accessible. An excitation coil 55 with the purpose to generate a homogeneous magnetic field at both sample containers surrounds measuring coils and sample containers, Excitation coil, measuring coils and also sample containers are placed concentric and also adjusted round the vertical center axis. Both respective position of the samples and also respective measuring coil can be adjusted separately. There is no need of an excitation coil when using the two last-mentioned, alternative detection methods.

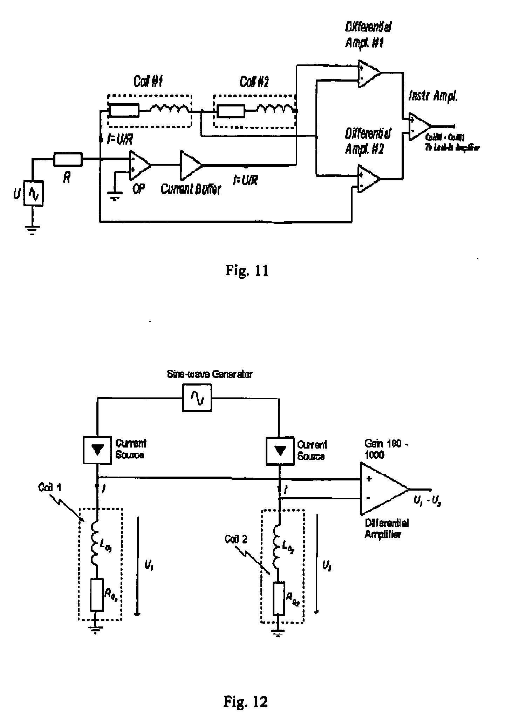

The measuring system 50 In Fig. 5 comprises a separate excitation coil 55. However, It is not necessary and can be excluded from the construction. Thus, the measuring system will be simpler as It will comprise only two coils, both being fed by identical AC-current an example of a similar coupling where the coils are coupled In parallel shown In Fig. 12. In this case, the excitation coils are removed from the measuring device 100 and only the coils 103 and 104 are used for both magnetization and detection.

The substantial advantages with the system of figure 5, not matter the excitation coil being removed or not, are partly the possibility of comparative measuring and partly the possibility of adjusting the system. The sensitivity of the system Is determined not only from the S/N state but also from the unbalance between two nominally Identical partial system containing sample container 1 (53) and sample container 2 (54) respectively with a detection coil each. The unbalance measured without sample container or with Identical sample container can occur for example as a result of:

- Slightly different number of revolutions In respective detection coil.

- In homogeneous magnetic field as a result of small tolerances when manufacturing concerning placing of samples In relation to the detection coil and excitation coil respectively.

- Different relative positions of the sample containers inside detection coils.

- Influence of manufacturing tolerances.

To reset (balance out) the difference In signal between the detection coils two methods can be used:

The system is constructed to make it possible to mechanically adjust position of respective sample container alternatively change the position of respective detection coil slightly so that unbalance in the difference signal is minimized.

The system Is however constructed to balance the difference signal in a faster and simpler way, by a determined amount of dry magnetic particles is provided in one of the spaces wherein the sample containers are placed (see Fig. 5 and 6). The particles create an extra contribution to the original signal that can be adjusted there through (set to zero). The dry magnetic particles do not show magnetic loss ($\chi'' = 0$) and also that the real part of the susceptibility Is constant ($\chi' =$ constant) In the examined frequency range.

There are a number of alternative detection methods:

Measuring coils as a feedback element in an oscillator circuit:

An alternative way of comparing two different preparations or modifications of the quantity of magnetic particles is to follow the thereby included frequency changes by means of a oscillator system wherein the detection coils constitutes the frequency determining element In an oscillator circuit, for example, in the return coil (feedback circuit) of the oscillator. it is well known that the resonance frequency of such an oscillator is $f_{max}$, while its coil factor is a measure of $\delta\, f_{max}$, which is a measure of the energy losses (friction) of the particles. When the detection coils constitutes the frequency determining elements in the circuit the resonance frequency will follow the changes of the L and R values of the coil, which is done the when the susceptibility of the particles is changed.

When detection of the AC difference between the coils is required, that is comparison of two different particle systems (or two different fluids) the coils surrounding respective sample are electrically phase shifted towards each other so that the resonance frequency is determined from the difference between the Inductance $\{\Delta L\ (= L_1 - L_2)\}$ and resistance $\{\Delta R\ (= R_1 - R_2)\}$ respectively of the coil. One way to accomplish this by means of only passive components is to place coils in an AC bridge. Active components, for example OP amplifiers, can also be used, which involves simple subtraction of two voltages from each other. A conception how a suitable coupling can be constructed, can be obtained by observing Fig. 12. The difference in each potential over the coils 103 and 104 is achieved by means of the amplifier 105.

If the output of the amplifier 105 is connected to the frequency generator 107 and the circuit is slightly modified, an oscillator is provided in which the coil is fed through the feedback through the current generators 106. Naturally, other circuit solution can occur, consequently the invention is not limited to the disclosed example.

The oscillator circuit can be shaped so that not only the frequency is detected but also changes In the total effect (or amplitude of the oscillators) to which the coil is exposed at different particle preparations: Frequency and dissipation will determine the <u>effective</u> changes of the circuit $\Delta L\ (= L_1 - L_2)$ and $\Delta R\ (= R_1 - R_2)$. These changes constitute a measure of changes of dissipation In the circuit. One can also determine an absolute measure of dissipation through measuring the subsiding of the oscillation when the coil is disconnected from the oscillator circuit.

Through detecting changes In oscillator frequency and also subsiding of signal amplitude from the oscillator system or effect changes (or amplitude changes) the response of the particles at a specific frequency, $f_{max}$ can be adjusted to the particle system used and also coil factor (energy losses) at the frequency can be determined.

The proceeding simplifies the measuring system when the need for a separate excitation coil vanishes.

*Measuring coils driven by means of a frequency generator*

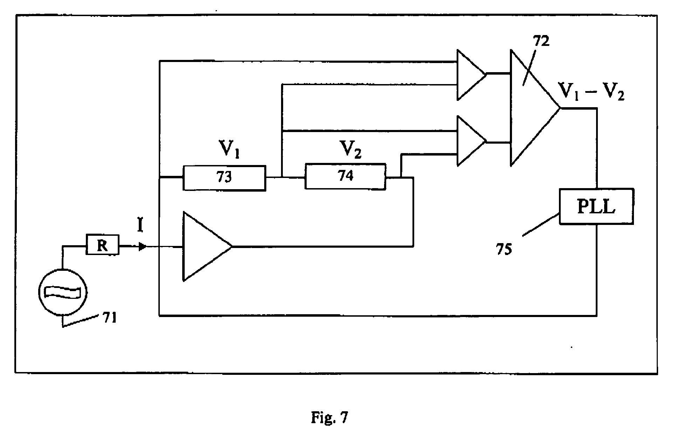

Another measuring principle for detecting the wanted voltage difference is constructed from phase lock (a so called Phase Lock Loop, PLL) according Fig. 7, showing a principle sketch over an alternating detection circuit 70, wherein the coils 73 and 74 are connected n series. A variable frequency generator alternatively a noise generator 71 is used, as input signal and also measure the complex voltage difference by means of a phase locked loop, The voltage difference is accomplished by means of a suitable connection of the operation amplifier 72. A similar effect can be obtained when constructing an AC bridge as well wherein two of the four branches of the bridge constitutes of coil 73 and coil 74 respectively. Theoretically, the voltage difference is determined out of phase with 0° and 90° respectively In relation to the input signal. In practice a certain extra phase displacement as a result of *operation amplifier.* Once again, detection of the signal difference at one and the same frequency between the two detection coils is desired.

A possible principle to accomplish the voltage difference according to the figure is by using an operation (instrument) amplifier in a suitable connection. Another possibility is based on placing respective coil in an AC bridge. The bridge is fed by an oscillator/frequency generator with a variable frequency at which the amplitude of the current floating through the coils is held constant.

The amplitude of the resulting voltage difference for a given phase displacement In relation to the input signal can be determined by means of a PLL circuit 75 (the phase difference Is proportional to a DC voltage determined/generated by the PLL circuit). Together with the measuring of the amplitude of the signal an enough description of the sample characteristics at a certain frequency is received. The advantages of the method Is above all being able to measure the magnetic characteristics of the particle system over a relatively broad frequency Interval and also that excitation coil isn't needed.

In addition, the coupling according to Fig. 12 can be used in a similar way as the one illustrated In Fig. 7. The output Is connected to a PLL-circuit, which senses the phase shift between the generated current from the current source 106 and the voltage difference at the output.

An alternative to using oscillator/frequency generator signals for generating time/period variable current is to excite the coils by means of white noise. The advantage is that one can receive frequency dependent Information through a FFT filtration of the response without using frequency generator.

The described sensor is a general analysis Instrument for analysis of different bio molecules or other molecules In fluid. Examples of molecules to be analyzed can for example be proteins In a fluid solution, such as blood, blood plasma, serum, and urine. The method function on condition that the analysis (molecule 2) can be connected to the particle in some way, for example through specific interaction with another molecule (molecule 1) that already before the beginning of the analysis has been connected to the particle, such as shown In Fig. 8. Observe that the dimensions (the size of the molecules in relation to the size of the particle) not are according to scale.

Yet, another alternative to the coupling of fig. 7 is illustrated In Fig. 11. In this coupling, a current buffer is arranged after the OP amplifier. The coils are denoted with "coil1" and "coil2". This coupling has the advantage of feeding two Identical currents through the coils.

Figs. 7 and 11 showed serially connected coils. In Fig. 12, the coils Coil1 and Coll2 are arranged in parallel. This allows a better measuring possibility where the signal-noise-ratio can be enhanced by directly measuring the differential signal from the two coils. The circuit comprises a sinus wave generator connected to two current sources. The output of each coil and also the input of each differential amplifier, the output signal of which is $U_1$-$U_2$, i.e. the voltage over the first and the second coil, respectively.

Since specific interactions are usually occurring In biological systems is it probably so that the sensor can get a distinguished role within this area, for example analysis of biochemical markers for different diseases, Examples of molecules that can Interact specific with each other are:

a) antibody - antigen

b) receptor - hormone

c) two complementary single strings of DNA

d) enzyme - substrate / enzyme - inhibitor

The particle system (for example particle size and choice of molecule 1) Is adapted according to size and type of molecule 2.

The sensor can for example be used within medical diagnostics. The new biosensor could for example be replacing some ELISA analysis (Enzyme Linked Immunsorbent Assay). This method is used today to a great extent to determine contents of biochemical markers (for example proteins) found in complex body fluids, such as blood, serum and cerebro-spinal fluid. Examples of ELISA analysis that can replace the new blosensor are:

a) analysis of tau proteins in cerebro-spinal fluid (part of diagnosis of Alzheimer's disease)
b) analysis of PSA in serum (diagnosis of prostate cancer)
c) analysis of acute phase proteins measured in connection with heart disease
d) analysis of CA 125 in serum (diagnosis of cancer in the ovaries)

It can be assumed that the sensor can be used fir detection of several markers at the same time through using particles with different sizes and/or different materials in the same system. The different particles are covered with different "blo molecule 1" (Fig. 8).

The new technique can be used for "low throughput screening", that Is the accomplishment of one or several analysis at the same time, or for "high throughput screening", that is the accomplishment of a large number of analysis simultaneously. The latter can be accomplished through multiply the sensor.

The invention is based on the use of magnetic particles. To make molecule 2 in the sample attach to the magnetic particle the surface of magnetic particle can be modified in a suitable way. This can be done for example through covering the surface of the particle with dextrane, with alkanethiols with suitable end groups, with certain peptides and so on. On the dextrane surface (or other suitable Intermediate layer) the molecule 1 can then, for example an antibody, be bond by means of for example cyanobromide activation or carboxyl acid activation. When molecule 1 Is connected to the magnetic particle the particles are mixed with a sample to be analyzed, for example serum.

To determine presence of blo molecules or antibodies In a carrier fluid containing magnetic particles with the suggested method, following steps must be accomplished In the sample preparation, measuring and analysis of measuring data.

1. Mixing the magnetic particles with the sample to be analyzed with respect to a certain substance.
2. Filling a sample container with the sample prepared according to point 1.
3. Placing a sample container In the detection coils or detection system (depending on which equipment used for measuring the frequency dependents of the magnetic response).
4. Applying an external measure field over the sample with a certain amplitude and frequency.
5. Measuring the magnetic response (both In phase and out of phase components) at this frequency.
6. Changing frequency and accomplishing a measurement according the points 4 and 5.
7. The analysis of the result Is to determine the Brownian relaxation period from in phase and out of phase components through using all data in the examined frequency Interval (up to about 10 kHz). An alternative analysis could be merely determining how large the frequency shift is (for the same value of In phase and out of phase components) at a couple of different frequencies.

The system allows a quantitative comparison between different fluid viscosities. The viscosity can be measured analogous with the thing described In the invention as to the rest with the difference that identical particle are used at viscosity measuring. Frequency changes occur as a result of different viscosities. It's not only the relaxation frequency, $f_{max}$, that will be changed but also $\delta f_{max}$. The advantage of the method compared with other ways of measuring the viscosity Is:

• relatively small fluid amounts is needed

• the possibility to measure viscosity locally round the particle, which make detection of viscosity gradients in a fluid volume possible

This viscosity detection method is however based on the particles still being stable In the different fluids.

REFERENCES

1. E. Kneller, In: Magnetism and Metallurgy vol. 1, eds. A.E. Berkowitz and E. Kneller, Academic Press New York (1969) 365.

2. C.P. Bean and J. Livingston, J. Appl. Phys. 30 (1959) 120S.

3. L. Néel, C.R. Acad. Scl. 228 (1949) 664.

4. Brown, W.F., 1963, J. Appl. Phys. 34, 1319.

5. Fannin, P.C., Scalfe, B.K.P. and Charles, S.W, 1988 J. Magn. Magn. Mater., 72, 95.

6. R. Kötltz, T. Bunte, W. Weitschies, L. Trahms, Superconducting quantum interference device-based magnetic nanoparticle relaxation measurement as a novel tool for the binding specific detection of biological binding reactions, J. Appl. Phys., 81, 8, 4317, 1997.

7. R. Kötitz, H. Matz, L. Trahms, H. Koch, W. Weltschies, T.Rheinlander, W. Semmler, T.Bunte, SQUID based remanence measurements for Immunoassays, IEEE Transactions on Applied Superconductivity, vol. 7, no. 2, 3678-81, 1997.

8. K. Enpuku, T. Minotanl, M. Hotta, A. Nakohado, Application of High Tc SQUID Magnetometer to Biological Immunoassays, IEEE Transactions on Applied Superconductivity, Vol. 11, No. 1, 661-664, 2001.

9. H. L. Grossman, Y. R. Chemla, Y. Poon, R. Stevens, J. Clarke, and M. D. Alper, Rapid, Sensitive, Selective Detection of Pathogenic Agents using a SQUID Microscope, Eurosensors XIV, 27-30, 2000.

10. Applications of Magnetic Particles In Immunoassays, Mary Meza, Ch.22 (pp.303-309) In "Scientific and Clinical Applications of Magnetic Carriers" ed. Häfell, et al. Plenum Press, New York, 1997; Lecture at conference in Rostock, Germany September 1996.

11. "The art of electronics", P. Horowitz and W. Hill, Cambridge Univ. Press, 2nd edition (1989).

12. "Design of crystal and other harmonic oscillators", B. Parzen, Wiley-Intersci Publ. (1983)

## Claims

1. Method for detecting changes of magnetic response of at least one magnetic particle provided with an external layer In a carrier fluid, wherein the method comprises using a measuring method comprising measuring the characteristic rotation time of said magnetic particle with respect to said external layer, which measuring method involves measuring Brownian relaxation in said carrier fluid under the influence of an external alternating magnetic field ***characterized by***
said method further involving that upon modification of the effective volume of the particle or its interaction with the carrier fluid a hydrodynamic volume of the particle changes, which implies a change of the frequency where an out of phase component of the magnetic susceptibility has its maximum.

2. Method according to claim 1,
   *characterized in*
   **that** said measuring involves measuring in-phase and/or out-phase components of a magnetic susceptibility in a frequency range.

3. Method according to claim 1,
   *characterized in*
   **that** the measurement comprises a relative measurement, whereby changes in a modified particle system are compared with an original system.

4. Method according to claim 3,
   *characterized in*
   **that** at least two sample containers and two detector coils are used.

5. Method according to claim 4,
   *characterized in*
   **that** an oscillator circuit Is used at first frequency, i.e. the resonant frequency, wherein detector coils are placed as a frequency determining element in the oscillating circuit so that they are out of phase with each other.

6. Method according to claim 5,
   *characterized in*
   **that** the frequency and/or an effect or amplitude of oscillations from the oscillating circuit over the coils is measured.

7. Method according to claim 4,
   *characterized in*
   **that** an external oscillator-/frequency generator is arranged, the coils are placed in and alternating bridge so that the difference between both detector coils Is measured, and that the phase difference between the out current and/or voltage of the frequency generator and a current/voltage over the bridge Is measured.

8. Method according to claim 7,
   *characterized in*
   **that** a difference in amplitude between the out current/voltage of the oscillator or frequency generator Is measured and compared with an amplitude of the current/voltage in the bridge.

9. Method according to claim 8,
   *characterized in*
   **that** the measurement is accomplished at one or several different frequencies.

10. Method according to claim 3,
    *characterized in*
    **that** a noise source is used and that the response of the system is analyzed by means of a Fast Fourier Transform, FFT, analysis of an outgoing signal.

11. Method according to claim 3,
    *characterized in*
    **that** a signal difference between said coils is set to zero.

12. Method according to claim 11,
    *characterized in*
    **that** said zero setting Is obtained through mechanically adjusting the position of each sample container alternatively changing the position each detector coil so that the signal difference is minimized.

13. Method according to claim 11,
    *characterized in*
    **that** said zero-setting is obtained through minimizing the signal by feeding a defined amount of a magnetic substance In one of the spaces comprising the sample containers, so that the substance creates an extra contribution to the original signal, which can be set to zero there through.

14. Method according to claim 13,
    *characterized in*
    **that** said magnetic substance shows substantially zero magnetic loss and that a real part of susceptibility is constant In the examined frequency range.

15. Method according to any of the claims 1-14,
    *characterized in*
    **that** the method Is used in the analysis instrument for analysis of different biomolecules or other molecules In a fluid.

16. Method according to claim 15,
    *characterized in*
    **that** said molecules, comprises one or several of proteins in a fluid solution, such as blood, blood plasma, serum and urine.

17. Method according to claim 15,
    *characterized in*
    **that** said analysis is connected to said particle through interaction with a second, which before the beginning of the analysis is connected to the particle.

18. Method according to claim 15,
    *characterized in*
    **that** molecules that specifically can be integrated with each other comprises one or several antibodies - antigen, receptors - hormone, two complementary single DNA strings and enzymes - substrate / enzyme - inhibitor.

19. Method according to any of the preceding claims,
    *characterized in*

**that** the surface of the magnetic particle is modified through covering the surface with one or several of dextranes, with alkanethiols, with suitable end-groups or with certain peptides.

20. Method according to claim 19,
    ***characterized in***
    **that** to a dextrane surface or other suitable intermediate layer can then a first molecule, for example an antibody, be bonded by means of for example cyanobromid activation or with carboxyl acid activation.

21. Device for detecting changes of magnetic response with at least one magnetic particle provided with an external layer in a carrier fluid, which method comprises measuring said magnetic particles characteristic rotation period regarding the effect of said external layer, wherein the measurement involves measurement of a Brownian relaxation in said carrier fluid under Influence of an external alternating magnetic field, said device comprising means for generating said alternating magnetic field, at least two substantially Identical detection coils connected to detection electronics and sample containers for absorbing carrier fluid,
    ***characterized in***
    **that** said device comprises means for measuring a change in the frequency, where an out of phase component of a magnetic susceptibility has its maximum at modification of the effective volume of the particle or its interaction with said carrier fluid when a hydrodynamic volume of said particle is changed.

22. Device of claim 21,
    ***characterized in***
    **that** said excitation coil surrounds detection coils and sample containers for generation of a homogeneous magnetic field by said sample container.

23. Device of claim 22,
    ***characterized in***
    **that** said excitation coil, measuring coils and sample containers are placed concentric and adjusted around their vertical center axis.

24. Device of claim 22,
    ***characterized in***
    **that** the device comprises a oscillator system wherein the detection coils forms a frequency determining element in an oscillator circuit.

25. Device of claim 21,
    ***characterized in***
    **that** said coils are arranged in the return coil of the oscillator.

26. Device of claim 21,
    ***characterized in***

**that** said coils are arranged as excitation coils.

27. Device of claim 21,
    ***characterized in***
    **that** the coils surrounding respective sample are electrical phase shifted versus each other so that the resonance frequency Is determined from the difference between the Inductance and resistance, respectively, of the coil.

28. Device of claim 21,
    ***characterized in***
    **that** the coils are placed in an AC-bridge.

29. Device claim 27,
    ***characterized In***
    **that** an op-amplifier is arranged for subtraction of two voltages from each other.

30. Device of claim 21,
    ***characterized in***
    **that** the device comprises a phase lock circuit.

31. Device of claim 21,
    ***characterized in***
    **that** the device comprises oscillator/frequency generator signals for generating time variable current for exciting the coils by means of white noise.

32. Device of claim 21,
    ***characterized in***
    **that** frequency depending information Is received through FFT-filtering of response.

33. Device of claim 21,
    ***characterized In***
    **that** the measurement over the coils is in series or parallel.

34. Device of claim 21,
    ***characterized in***
    **that** the device comprises a circuit comprising a sinus wave generator connected to two current sources, the output of each current source being connected to each one end of each coil and also the input of a differential amplifier, having an output being the voltage difference over the first and the second coils.

35. Device of claim 21,
    ***characterized in***
    **that** the device further comprises a noise source and that the response of the device Is analyzed using a Fast Fourier Transform, FFT, analyze of one of output signals.

36. Method for determining an amount of molecules In a carrier fluid containing magnetic particles using a method for detecting changes of a magnetic response according to claim 1, the method comprising the steps of:

A. providing particles with a layer, which inter-/reacts with the substance to be analyzed,

B. mixing the magnetic particles with the sample to be analyzed with respect to molecules,

C. filling a sample container with fluid being prepared according to B,

D. placing a sample container in the detection system,

E. applying an external measurement field over the sample with a certain amplitude and frequency,

F. measuring the magnetic response at this frequency,

G. changing frequency and performing measurement again according to D and E,

H. analyzing the result through determining a Brownian relaxation time from in phase and out of phase components by using data in the examined frequency Interval.

**37.** Method of claim 36,
*characterized in*
determining the frequency shift for the same value of in phase and out of phase component at different frequencies.

**38.** Method of claim 36 or 37,
*characterized in*
**that** said molecules consist of a biomolecule.

**Patentansprüche**

**1.** Verfahren zum Erfassen von Veränderungen der magnetischen Antwort wenigstens eines mit einer externen Schicht versehenen magnetischen Partikels in einem Trägerfluid, wobei das Verfahren eine Verwendung eines Messverfahrens umfasst, das ein Messen der charakteristischen Rotationszeit des magnetischen Partikels mit Bezug auf die externe Schicht umfasst, wobei das Messverfahren ein Messen der Brownschen Relaxation in dem Trägerfluid unter dem Einfluss eines externen Wechselmagnetfeldes einschließt,
*dadurch gekennzeichnet,*
**dass** das Verfahren weiterhin einschließt, dass sich bei Modifikation des effektiven Volumens des Partikels oder seiner Wechselwirkung mit dem Trägerfluid ein hydrodynamisches Volumen des Partikels verändert, was eine Veränderung der Frequenz impliziert, bei der eine außerphasige Komponente der magnetischen Suszeptibilität ihr Maximum hat.

**2.** Verfahren gemäß Anspruch 1,
*dadurch gekennzeichnet,*

**dass** das Messen ein Messen von phasengleichen und/oder außerphasigen Komponenten einer magnetischen Suszeptibilität in einem Frequenzbereich einschließt.

**3.** Verfahren gemäß Anspruch 1,
*dadurch gekennzeichnet,*
dass die Messung eine relative Messung umfasst, durch die Veränderungen in einem modifizierten Partikelsystem mit einem ursprünglichen System verglichen werden.

**4.** Verfahren gemäß Anspruch 3,
*dadurch gekennzeichnet,*
**dass** wenigstens zwei Probenbehälter und zwei Detektorspulen verwendet werden.

**5.** Verfahren gemäß Anspruch 4,
*dadurch gekennzeichnet,*
**dass** eine Oszillatorschaltung bei einer ersten Frequenz, d.h. der Resonanzfrequenz, verwendet wird, wobei Detektorspulen so als ein Frequenzbestimmungselement in der Oszillationsschaltung platziert sind, dass sie zueinander außerphasig sind.

**6.** Verfahren gemäß Anspruch 5,
*dadurch gekennzeichnet,*
**dass** die Frequenz und/oder eine Wirkung oder Amplitude von Oszillationen aus der Oszillationsschaltung über die Spulen gemessen wird.

**7.** Verfahren gemäß Anspruch 4,
*dadurch gekenntzeichnet,*
dass ein externer Oszillator-/Frequenzgenerator angeordnet ist, die Spulen in einer Wechselspannungsbrücke platziert sind, so dass die Differenz zwischen beiden Detektorspulen gemessen wird, und dass die Phasendifferenz zwischen dem Ausgangsstrom und/oder der Ausgangsspannung des Frequenzgenerators und einem Strom / einer Spannung über die Brücke gemessen wird.

**8.** Verfahren gemäß Anspruch 7,
*dadurch gekennzeichnet,*
**dass** eine Differenz der Amplitude zwischen dem Ausgangsstrom / der Ausgangsspannung des Oszillators oder Frequenzgenerators gemessen wird und mit einer Amplitude des Stroms / der Spannung in der Brücke verglichen wird.

**9.** Verfahren gemäß Anspruch 8,
*dadurch gekennzeichnet,*
**dass** die Messung bei einer oder mehreren unterschiedlichen Frequenzen bewerkstelligt wird.

**10.** Verfahren gemäß Anspruch 3,
*dadurch gekennzeichnet,*
**dass** eine Rauschquelle verwendet wird und dass die Antwort des Systems vermittels einer Schnelle-Fourier-Transformations-, FFT-, Analyse eines ausgehenden Signals analysiert wird.

11. Verfahren gemäß Anspruch 3,
    ***dadurch gekennzeichnet,***
    **dass** eine Signaldifferenz zwischen den Spulen auf null gesetzt wird.

12. Verfahren gemäß Anspruch 11,
    ***dadurch gekennzeichnet,***
    **dass** die Nullsetzung durch ein mechanisches Justieren der Position jedes Probenbehälters erzielt wird, wobei alternativ die Position jeder Detektorspule so verändert wird, dass die Signaldifferenz minimiert wird.

13. Verfahren gemäß Anspruch 11,
    ***dadurch gekennzeichnet,***
    **dass** die Nullsetzung durch ein Minimieren des Signals erzielt wird, indem eine definierte Menge einer magnetischen Substanz in einen der Räume eingeführt wird, die die Probenbehälter umfassen, so dass die Substanz einen zusätzlichen Beitrag zu dem ursprünglichen Signal erzeugt, das dadurch auf null gesetzt werden kann.

14. Verfahren gemäß Anspruch 13,
    ***dadurch gekennzeichnet,***
    **dass** die magnetische Substanz einen magnetischen Verlust von im Wesentlichen null aufweist und dass ein Realteil der Suszeptibilität in dem untersuchten Frequenzbereich konstant ist.

15. Verfahren gemäß einem der Ansprüche 1 - 14,
    ***dadurch gekennzeichnet,***
    **dass** das Verfahren in dem Analyseinstrument zur Analyse unterschiedlicher Biomoleküle oder anderer Moleküle in einem Fluid verwendet wird.

16. Verfahren gemäß Anspruch 15,
    ***dadurch gekennzeichnet,***
    **dass** die Moleküle ein oder mehrere Proteine in einer Fluidlösung wie etwa Blut, Blutplasma, Serum und Urin umfassen.

17. Verfahren gemäß Anspruch 15,
    ***dadurch gekennzeichnet,***
    **dass** die Analyse mit dem Partikel durch Wechselwirkung mit einem zweiten verbunden ist, das vor Beginn der Analyse mit dem Partikel verbunden ist.

18. Verfahren gemäß Anspruch 15,
    ***dadurch gekennzeichnet,***
    **dass** Moleküle, die spezifisch miteinander integriert sein können, eines oder mehrere von Antikörper-Antigen, Rezeptoren-Hormon, zwei komplementären einzelnen DNA-Ketten und Enzyme-Substrat / Enzym-Inhibitor umfassen.

19. Verfahren gemäß einem der vorhergehenden Ansprüche,
    ***dadurch gekennzeichnet,***
    **dass** die Oberfläche des magnetischen Partikels durch Bedecken der Oberfläche mit einem oder mehreren von Dextranen, mit Alkanthiolen, mit geeigneten Endgruppen oder mit bestimmten Peptiden modifiziert wird.

20. Verfahren gemäß Anspruch 19,
    ***dadurch gekennzeichnet,***
    **dass** an eine Dextranoberfläche oder andere geeignete Zwischenschicht dann ein erstes Molekül, beispielsweise ein Antikörper, vermittels von beispielsweise Bromcyanaktivierung oder mit Carbonsäureaktivierung gebunden werden kann.

21. Vorrichtung zum Erfassen von Veränderungen der magnetischen Antwort bei wenigstens einem mit einer externen Schicht versehenen magnetischen Partikel in einem Trägerfluid, wobei das Verfahren ein Messen der charakteristischen Rotationsperiode des magnetischen Partikels hinsichtlich der Wirkung der externen Schicht umfasst, wobei die Messung eine Messung einer Brownschen Relaxation in dem Trägerfluid unter dem Einfluss eines externen Wechselmagnetfeldes einschließt, wobei die Vorrichtung Mittel zum Erzeugen des Wechselmagnetfeldes, wenigstens zwei im Wesentlichen identische Detektionsspulen, die mit Detektionselektronik verbunden sind, und Probenbehälter zum Aufnehmen von Trägerfluid umfasst,
    ***dadurch gekennzeichnet,***
    **dass** die Vorrichtung Mittel zum Messen einer Veränderung der Frequenz umfasst, wobei eine außerphasige Komponente einer magnetischen Suszeptibilität ihr Maximum bei einer Modifikation des effektiven Volumens des Partikels oder seiner Wechselwirkung mit dem Trägerfluid hat, wenn ein hydrodynamisches Volumen des Partikels verändert wird.

22. Vorrichtung gemäß Anspruch 21,
    ***dadurch gekennzeichnet,***
    **dass** die Erregerspule Detektionsspulen und Probenbehälter zur Erzeugung eines homogenen Magnetfeldes durch den Probenbehälter umgibt.

23. Vorrichtung gemäß Anspruch 22,
    ***dadurch gekennzeichnet,***
    **dass** die Erregerspule, die Messspulen und die Probenbehälter konzentrisch platziert und um ihre vertikale Mittelachse justiert sind.

24. Vorrichtung gemäß Anspruch 22,
    ***dadurch gekennzeichnet,***
    **dass** die Vorrichtung ein Oszillatorsystem umfasst, wobei die Detektionsspulen ein Frequenzbestimmungselement in einer Oszillatorschaltung bilden.

**25.** Vorrichtung gemäß Anspruch 21,
*dadurch gekennzeichnet,*
**dass** die Spulen in der Rückführspule des Oszillators angeordnet sind.

**26.** Vorrichtung gemäß Anspruch 21,
*dadurch gekennzeichnet,*
**dass** die Spulen als Erregerspulen angeordnet sind.

**27.** Vorrichtung gemäß Anspruch 21,
*dadurch gekennzeichnet,*
**dass** die Spulen, die die jeweilige Probe umgeben, gegeneinander in der elektrischen Phase verschoben sind, so dass die Resonanzfrequenz aus der Differenz zwischen der Induktanz beziehungsweise dem Widerstand der Spule bestimmt wird.

**28.** Vorrichtung gemäß Anspruch 21,
*dadurch gekennzeichnet,*
**dass** die Spulen in einer Wechselspannungsbrücke platziert sind.

**29.** Vorrichtung gemäß Anspruch 27,
*dadurch gekennzeichnet,*
**dass** ein Operationsverstärker zur Subtraktion zweier Spannungen voneinander angeordnet ist.

**30.** Vorrichtung gemäß Anspruch 21,
*dadurch gekennzeichnet,*
**dass** die Vorrichtung eine Phasenregelschaltung umfasst.

**31.** Vorrichtung gemäß Anspruch 21,
*dadurch gekennzeichnet,*
**dass** die Vorrichtung Oszillator-/Frequenzgeneratorsignale zum Generieren von zeitvariablem Strom zum Erregen der Spulen vermittels weißen Rauschens umfasst.

**32.** Vorrichtung gemäß Anspruch 21,
*dadurch gekennzeichnet,*
**dass** frequenzabhängige Informationen durch eine FFT-Filterung der Antwort erhalten werden.

**33.** Vorrichtung gemäß Anspruch 21,
*dadurch gekennzeichnet,*
**dass** die Messung über die Spulen seriell oder parallel ist.

**34.** Vorrichtung gemäß Anspruch 21,
*dadurch gekennzeichnet,*
**dass** die Vorrichtung eine Schaltung umfasst, die einen Sinuswellengenerator umfasst, der mit zwei Stromquellen verbunden ist, wobei der Ausgang jeder Stromquelle jeweils mit einem Ende jeder Spule und auch dem Eingang eines Differenzverstärkers verbunden ist, der einen Ausgang hat, welcher die Spannungsdifferenz über die ersten und zweiten Spulen ist.

**35.** Vorrichtung gemäß Anspruch 21,
*dadurch gekennzeichnet,*
**dass** die Vorrichtung weiterhin eine Rauschquelle umfasst und dass die Antwort der Vorrichtung unter Verwendung einer Schnelle-Fourier-Transformations-, FFT-, Analyse eines der Ausgangssignale analysiert wird.

**36.** Verfahren zum Bestimmen einer Menge von Molekülen in einem Trägerfluid, das magnetische Partikel enthält, unter Verwendung eines Verfahrens zum Erfassen von Veränderungen einer magnetischen Antwort gemäß Anspruch 1, wobei das Verfahren die Schritte umfasst:

A. Versehen von Partikeln mit einer Schicht, die mit der zu analysierenden Substanz wechselwirkt / reagiert,
B. Mischen der magnetischen Partikel mit der mit Bezug auf Moleküle zu analysierenden Probe,
C. Füllen eines Probenbehälters mit Fluid, das gemäß B präpariert ist,
D. Anordnen eines Probenbehälters in dem Detektionssystem,
E. Anlegen eines externen Messfeldes über der Probe mit einer bestimmten Amplitude und Frequenz,
F. Messen der magnetischen Antwort bei dieser Frequenz,
G. Verändern der Frequenz und erneutes Durchführen einer Messung gemäß D und E,
H. Analysieren des Ergebnisses durch Bestimmen einer Brownschen Relaxationszeit aus phasengleichen und außerphasigen Komponenten durch Verwendung von Daten in dem untersuchten Frequenzintervall.

**37.** Verfahren gemäß Anspruch 36,
*gekennzeichnet durch*
Bestimmen der Frequenzverschiebung für denselben Wert von phasengleichen und außerphasigen Komponenten bei unterschiedlichen Frequenzen.

**38.** Verfahren gemäß Anspruch 36 oder 37,
*dadurch gekennzeichnet,*
**dass** die Moleküle aus einem Biomolekül bestehen.

**Revendications**

**1.** Procédé, destiné à détecter des changements de réponse magnétique d'au moins une particule magnétique, munie d'une couche extérieure dans un fluide porteur, dans lequel le procédé comprend l'opération, consistant à utiliser un procédé de mesure, comprenant la mesure du temps caractéristique de rotation de ladite particule magnétique par rapport à ladite couche extérieure, lequel procédé de mesure implique de mesurer la relaxation de Brown dans ledit fluide porteur, sous l'influence d'un champ magnétique alternatif extérieur, **caractérisé en ce**
**que** ledit procédé suppose, en outre, le fait qu'en cas de modification du volume effectif de la particule ou de son interaction avec le fluide porteur, un volume hydrodynamique de la particule change, ce qui implique un changement de la fréquence, là où une composante déphasée de la susceptibilité magnétique a son maximum.

2. Procédé selon la revendication 1, **caractérisé en ce**
**que** ladite mesure suppose la mesure de composantes en phase et / ou déphasées d'une susceptibilité magnétique, dans une plage de fréquences.

3. Procédé selon la revendication 1, **caractérisé en ce**
**que** la mesure comprend une mesure relative, moyennant quoi les changements dans un système modifié de particules sont comparés avec un système original.

4. Procédé selon la revendication 3, **caractérisé en ce**
**qu'**au moins deux conteneurs échantillons et deux bobines de détecteur sont utilisés.

5. Procédé selon la revendication 4, **caractérisé en ce**
**qu'**un circuit d'oscillation est utilisé à une première fréquence, c'est-à-dire la fréquence de résonance, dans lequel les bobines de détecteur sont placées en tant qu'élément déterminant la fréquence dans le circuit d'oscillation, de sorte qu'elles sont déphasées l'une par rapport à l'autre.

6. Procédé selon la revendication 5, **caractérisé en ce**
**que** la fréquence et / ou un effet ou une amplitude d'oscillations par le circuit oscillant sur les bobines est / sont mesuré(e) (s).

7. Procédé selon la revendication 4, **caractérisé en ce**
**qu'**un générateur d'oscillation / de fréquence est agencé, que les bobines sont placées dans un pont alternatif, de sorte que la différence entre les deux bobines de détecteur est mesurée et que la différence de phase entre le courant et / ou la tension coupé(e) du générateur de fréquence et un courant / une tension sur le pont est mesurée.

8. Procédé selon la revendication 7, **caractérisé en ce**
**qu'**une différence d'amplitude entre le courant / la tension coupé(e) du générateur d'oscillations ou de fréquences est mesurée et comparée avec une amplitude du courant / de la tension dans le pont.

9. Procédé selon la revendication 8, **caractérisé en ce**
**que** la mesure est effectuée à une ou plusieurs fréquences différentes.

10. Procédé selon la revendication 3, **caractérisé en ce**
**qu'**une source de bruit est utilisée et que la réponse du système est analysée au moyen d'une transformée de Fourier rapide, TFR, d'une analyse d'un signal de départ.

11. Procédé selon la revendication 3, **caractérisé en ce**
**qu'**une différence de signal entre lesdites bobines est mise à zéro.

12. Procédé selon la revendication 11, **caractérisé en ce**
**que** ladite mise à zéro est obtenue en ajustant mécaniquement la position de chaque conteneur échantillon, en changeant alternativement la position de chaque bobine de détecteur, de sorte que la différence de signaux est minimisée.

13. Procédé selon la revendication 11, **caractérisé en ce**
**que** ladite mise à zéro est obtenue en diminuant le signal, en amenant une quantité définie de substance magnétique dans l'un des espaces comprenant les conteneurs échantillons, de sorte que la substance crée une contribution spéciale au signal original, qui peut être mise à zéro à travers celui-ci.

14. Procédé selon la revendication 13, **caractérisé en ce**
**que** ladite substance magnétique affiche sensiblement une perte magnétique nulle et qu'une part réelle de susceptibilité est constante dans la plage examinée de fréquences.

15. Procédé selon l'une quelconque des revendications 1 - 14, **caractérisé en ce**
**que** le procédé est utilisé dans l'instrument d'analyse, pour analyser différentes biomolécules ou d'autres molécules dans un fluide.

**16.** Procédé selon la revendication 15, **caractérisé en ce que** lesdites molécules comprennent une ou plusieurs protéines dans une solution fluidique, telle que le sang, le plasma sanguin, le sérum et l'urine.

**17.** Procédé selon la revendication 15, **caractérisé en ce que** ladite analyse est reliée à ladite particule par interaction avec une deuxième analyse, qui est reliée à la particule, avant le début de l'analyse.

**18.** Procédé selon la revendication 15, **caractérisé en ce que** les molécules, qui peuvent être intégrées spécifiquement entre elles, comprennent une ou plusieurs anticorps - antigène, récepteurs - hormone, deux séries et enzymes séparés complémentaires d'ADN - substrat / enzyme - inhibiteur.

**19.** Procédé selon l'une quelconque des revendications précédentes, **caractérisé en ce que** la surface de la particule magnétique est modifiée par recouvrement de la surface avec un ou plusieurs dextranes, avec des alkanéthiols, avec des groupes terminaux appropriés ou avec certains peptides.

**20.** Procédé selon la revendication 19, **caractérisé en ce que**, sur une surface revêtue de dextrane ou autre couche intermédiaire appropriée, une première molécule, par exemple un anticorps, peut, alors, être liée au moyen, par exemple, d'une activation au cyanobromure ou avec une activation à l'acide carboxylique.

**21.** Dispositif, destiné à détecter des changements de réponse magnétique avec au moins une particule magnétique, munie d'une couche extérieure dans un fluide porteur, ledit procédé comprenant l'opération, consistant à mesurer ladite période de rotation, caractéristique des particules magnétiques, en ce qui concerne l'effet de ladite couche extérieure, dans lequel la mesure implique de mesurer une relaxation de Brown dans ledit fluide porteur, sous l'influence d'un champ magnétique alternatif extérieur, ledit dispositif comprenant un moyen, destiné à générer ledit champ magnétique alternatif, au moins deux bobines de détection, sensiblement identiques, connectées à un système électronique de détection et des conteneurs échantillons, destinés à absorber le fluide porteur, **caractérisé en ce que** ledit dispositif comprend un moyen, destiné à mesurer un changement dans la fréquence, où une composante déphasée d'une susceptibilité magnétique a son maximum, lors de la modification du volume effectif de la particule ou de son interaction avec ledit fluide porteur, lorsqu'un volume hydrodynamique de ladite particule est changé.

**22.** Dispositif selon la revendication 21, **caractérisé en ce que** ladite bobine d'excitation entoure les bobines de détection et les conteneurs échantillons pour la création d'un champ magnétique homogène par ledit conteneur échantillon.

**23.** Dispositif selon la revendication 22, **caractérisé en ce que** ladite bobine d'excitation, lesdites bobines de mesure et lesdits conteneurs échantillons sont placés de manière concentrique et ajustés autour de leur axe médian vertical.

**24.** Dispositif selon la revendication 22, **caractérisé en ce que** le dispositif comprend un système d'oscillation, dans lequel les bobines de détection forment un élément, déterminant la fréquence, dans un circuit d'oscillations.

**25.** Dispositif selon la revendication 21, **caractérisé en ce que** lesdites bobines sont agencées dans la bobine de retour de l'oscillateur.

**26.** Dispositif selon la revendication 21, caractérisé en ce lesdites bobines sont agencées en tant que bobines d'excitation.

**27.** Dispositif selon la revendication 21, **caractérisé en ce que** les bobines, qui entourent l'échantillon respectif, subissent un décalage de phases électriques l'une envers l'autre, de sorte que la fréquence de résonance est déterminée à partir de la différence entre l'inductance et la résistance, respectivement, de la bobine.

**28.** Dispositif selon la revendication 21, **caractérisé en ce que** les bobines sont placées dans un pont CA.

**29.** Dispositif selon la revendication 27, **caractérisé en ce qu'**un amplificateur opérationnel est agencé, pour la soustraction de deux tensions l'une de l'autre.

**30.** Dispositif selon la revendication 21, **caractérisé en ce que** le dispositif comprend un circuit de verrouillage de phase.

**31.** Dispositif selon la revendication 21, **caractérisé en ce que** le dispositif comprend des signaux d'un générateur d'oscillations / de fréquences, destinés à générer un courant, variable dans le temps, destiné à exciter les bobines au moyen d'un bruit blanc.

**32.** Dispositif selon la revendication 21, **caractérisé en ce que** l'information, fonction de la fréquence, est reçue par filtrage TFR de la réponse.

**33.** Dispositif selon la revendication 21, **caractérisé en ce que** la mesure sur les bobines est opérée en série ou en parallèle.

**34.** Dispositif selon la revendication 21, **caractérisé en ce que** le dispositif comprend un circuit, comprenant un générateur d'ondes sinusoïdales, connecté à deux sources de courant, la sortie de chaque source de courant étant connectée à chaque extrémité de chaque bobine et, également, à l'entrée d'un amplificateur différentiel, ayant une sortie qui représente la différence de tension sur la première et la deuxième bobine.

**35.** Dispositif selon la revendication 21, **caractérisé en ce que** le dispositif comprend, en outre, une source de bruits et que la réponse du dispositif est analysée en utilisant une transformée de Fourier rapide, TFR, une analyse de l'un des signaux de sortie.

**36.** Procédé, destiné à déterminer une quantité de molécules dans un fluide porteur, contenant des particules magnétiques, en utilisant un procédé, destiné à détecter des changements d'une réponse magnétique selon la revendication 1, le procédé comprenant les étapes, consistant à :

    A. munir les particules d'une couche, qui inter / réagit avec la substance à analyser ;
    B. mélanger les particules magnétiques avec l'échantillon à analyser, par rapport aux molécules ;
    C. remplir un conteneur échantillon avec du fluide, préparé selon B ;
    D. placer un conteneur échantillon dans le système de détection ;
    E. appliquer un champ extérieur de mesure sur l'échantillon avec une certaine amplitude et une certaine fréquence ;
    F. mesurer la réponse magnétique à cette fréquence ;
    G. changer la fréquence et exécuter à nouveau une mesure selon D et E ;

    H. analyser le résultat en déterminant un temps de relaxation de Brown, à partir de composantes en phase et déphasées, en utilisant des données dans l'intervalle examiné de fréquences.

**37.** Procédé selon la revendication 36, caractérisé en ce ce qu'il détermine le décalage de fréquences pour la même valeur de composante en phase et déphasée, à des fréquences différentes.

**38.** Dispositif selon la revendication 36 ou 37, **caractérisé en ce que** lesdites molécules consistent en une biomolécule.

Fig. 1

Fig. 2

Fig. 3

Fig. 4

Fig. 5

Fig. 6

Fig. 7

Molecule 2

Molecule 1

Magnetic particle

Fig. 8

Monoclonal antibody

Epitop 2

Antigen

Epitop 1

Epitop 3

Fig. 9

Fig. 10

Fig. 11

Fig. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6027946 A

**Non-patent literature cited in the description**

- **E. KNELLER.** Magnetism and Metallurgy. Academic Press, 1969, vol. 1, 365
- **C.P. BEAN ; J. LIVINGSTON.** *J. Appl. Phys.,* 1959, vol. 30, 120S
- **L. NÉEL.** *C.R. Acad. Scl.,* 1949, vol. 228, 664
- **BROWN, W.F.** *J. Appl. Phys.,* 1963, vol. 34, 1319
- **FANNIN, P.C. ; SCALFE, B.K.P. ; CHARLES, S.W.** *J. Magn. Magn. Mater.,* 1988, vol. 72, 95
- **R. KÖTLTZ ; T. BUNTE ; W. WEITSCHIES ; L. TRAHMS.** Superconducting quantum interference device-based magnetic nanoparticle relaxation measurement as a novel tool for the binding specific detection of biological binding reactions. *J. Appl. Phys.,* 1997, vol. 81 (8), 4317
- **R. KÖTITZ ; H. MATZ ; L. TRAHMS ; H. KOCH ; W. WELTSCHIES ; T.RHEINLANDER ; W. SEMMLER ; T. BUNTE.** SQUID based remanence measurements for Immunoassays. *IEEE Transactions on Applied Superconductivity,* 1997, vol. 7 (2), 3678-81

- **K. ENPUKU ; T. MINOTANL ; M. HOTTA ; A. NA-KOHADO.** Application of High Tc SQUID Magnetometer to Biological Immunoassays. *IEEE Transactions on Applied Superconductivity,* 2001, vol. 11 (1), 661-664
- **H. L. GROSSMAN ; Y. R. CHEMLA ; Y. POON ; R. STEVENS ; J. CLARKE ; M. D. ALPER.** Rapid, Sensitive, Selective Detection of Pathogenic Agents using a SQUID Microscope. *Eurosensors XIV,* 2000, 27-30
- Applications of Magnetic Particles In Immunoassays. **MARY MEZA et al.** Scientific and Clinical Applications of Magnetic Carriers. Plenum Press, 1997, 303-309
- *Lecture at conference in Rostock,* 1996
- **P. HOROWITZ ; W. HILL.** The art of electronics. Cambridge Univ. Press, 1989
- **B. PARZEN.** Design of crystal and other harmonic oscillators. Wiley-Intersci Publ, 1983